Europäisches Patentamt

(19) European Patent Office

Office européen des brevets

(11) Numéro de publication : **0 076 740**
**B1**

(12) **FASCICULE DE BREVET EUROPÉEN**

(45) Date de publication du fascicule du brevet :
02.01.86

(21) Numéro de dépôt : **82401760.2**

(22) Date de dépôt : **28.09.82**

(51) Int. Cl.⁴ : **A 61 F   9/00**

(54) **Appareil de chirurgie ophtalmologique à laser.**

(30) Priorité : **02.10.81 FR 8118612**

(43) Date de publication de la demande :
**13.04.83 Bulletin 83/15**

(45) Mention de la délivrance du brevet :
**02.01.86 Bulletin 86/01**

(84) Etats contractants désignés :
**AT BE CH DE GB IT LI LU NL SE**

(56) Documents cités :
**EP-A- 0 007 256**
**EP-A- 0 030 210**
**US-A- 3 710 798**
**US-A- 3 851 974**
**US-A- 4 079 230**
**MUTZE: "ABC der Optic", 1961, pages 180 et 26,**
**Verlag Werner Dausien, Hanau/Main, DE**

(73) Titulaire : **SYNTHELABO**
**58, rue de la Glacière**
**F-75621 Paris Cedex 13 (FR)**

(72) Inventeur : **Tagnon, Luc**
**11 Rue Brière de Boismont**
**F-94160 Saint Mande (FR)**

(74) Mandataire : **CABINET BONNET-THIRION**
**95 Boulevard Beaumarchais**
**F-75003 Paris (FR)**

Jouve, 18, rue St-Denis, 75001 Paris, France

**Description**

La présente invention concerne d'une manière générale les appareils de chirurgie ophtalmologique mettant en œuvre un laser, ou laser opératoire, propre à assurer une coupure des tissus à traiter sans échauffement de ceux-ci.

La génération post-opératoire de tissus cicatriciels peut ainsi être avantageusement évitée.

La puissance du flux lumineux véhiculé par le faisceau du laser opératoire à mettre en œuvre doit en pratique être suffisamment élevée pour que l'intervention de ce faisceau se fasse non pas par transfert thermique, mais par claquage optique, c'est-à-dire par dissociation atomique des tissus visés.

Outre l'absence d'échauffement pour ceux-ci, il en résulte avantageusement la formation locale d'un plasma de densité électronique suffisante pour être opaque au rayonnement qui lui a donné naissance, en sorte que les tissus en aval, et notamment la rétine, sont avantageusement protégés par ce rayonnement.

Pour que la puissance du flux lumineux véhiculé par le faisceau du laser opératoire mis en œuvre ait la valeur élevée recherchée, il faut que les impulsions de ce faisceau soient ultra-brèves, de l'ordre maximum de quelques dizaines de picosecondes.

Un laser YAG en mode bloqué émettant dans l'infrarouge est à cet égard bien adapté.

Pour matérialiser pour l'opérateur le point de visée d'un tel laser opératoire à faisceau infrarouge, ce qui est nécessaire pour en situer l'intervention de manière très exacte sur les tissus à traiter, il est usuel d'associer à ce laser opératoire, ou laser principal, un laser auxiliaire continu à faisceau visible de faible puissance formant laser de visée.

Un appareil de chirurgie ophtalmologique comportant ainsi un laser principal opératoire à faisceau infrarouge et un laser auxiliaire de visée à faisceau visible se trouve notamment décrit dans la demande de brevet européen déposée le 7 Juin 1979 sous le N° 79400370.7 et publiée sous le N° 0.007.256.

En pratique, un jeu de miroirs est mis en œuvre pour superposer les faisceaux des deux lasers à l'entrée d'une optique d'exploitation, et celle-ci, essentiellement formée d'une lampe à fente, comporte, entre l'unité à lasers et la dite lampe à fente, d'une part, un bras articulé de transfert propre à assurer la connexion souhaitée entre ces deux éléments, et d'autre part, une lentille de focalisation, propre à assurer la concentration requise des faisceaux des lasers au point opératoire.

En pratique, à chacun des lasers il est associé, immédiatement en aval de celui-ci, un dispositif optique, communément dit « expandeur de faisceau », ou « afocal », propre à en augmenter le diamètre du faisceau pour en réduire la densité superficielle de flux lumineux dans un plan de section droite.

La raison en est triple.

Il faut, tout d'abord, obtenir une bonne localisation du point de traitement par une convergence aussi élevée que possible du faisceau infrarouge pénétrant dans l'œil.

Il faut également éviter une traumatisation inutile des tissus traversés en amont du point opératoire et notamment de l'épithelium de la cornée, en réduisant la densité de puissance dans la section du faisceau infrarouge à l'endroit de ces traversées.

Il faut, enfin, éviter l'endommagement des miroirs mis en œuvre pour assurer la superposition des faisceaux des deux lasers, qui sont usuellement des miroirs métalliques classiques.

Un tel expandeur de faisceau est au moins formé d'une lentille d'entrée et d'une lentille de sortie.

Dans l'appareil de chirurgie ophtalmologique décrit dans la demande de brevet européen mentionnée ci-dessus, les expandeurs de faisceau mis en œuvre, qui doivent être au moins en partie distincts l'un de l'autre en raison de la nécessité qu'il y a de traiter séparément les deux faisceaux des lasers compte tenu des longueurs d'onde différentes de ceux-ci, sont totalement distincts l'un de l'autre.

Il en résulte que, pour chacun de ces expandeurs de faisceau, les lentilles d'entrée et de sortie sont très proches l'une de l'autre.

Compte tenu du rapport d'expansion à assurer, en pratique de l'ordre de dix, leurs focales sont donc relativement courtes, et, partant, leurs puissances relativement élevées, de l'ordre de 40 dioptries en pratique pour la lentille d'entrée.

Il s'agit donc inévitablement de lentilles de relativement petits diamètres d'ouverture, qui sont particulièrement difficiles à réaliser et à centrer.

La présente invention a d'une manière générale pour objet une disposition permettant d'éviter cet inconvénient et conduisant en outre à d'autres avantages.

De manière plus précise, elle a pour objet un appareil de chirurgie ophtalmologique du genre comportant un laser principal à faisceau infrarouge, ou laser opératoire, un laser auxiliaire à faisceau visible, ou laser de visée, un jeu de miroirs propres à superposer lesdits faisceaux à l'entrée d'une optique d'exploitation, et, pour chacun de ceux-ci, un expandeur de faisceau au moins formé d'une lentille d'entrée et d'une lentille de sortie, cet appareil de chirurgie ophtalmologique étant caractérisé en ce que l'expandeur de faisceau du laser à faisceau infrarouge et l'expandeur de faisceau du laser à faisceau visible ont en commun leur lentille de sortie.

Grâce à une telle disposition, la lentille de sortie ainsi en commun entre les deux expandeurs de faisceau, qui intéresse le faisceau de chacun des lasers, peut être disposée aussi près que souhaité de l'optique d'utilisation, et en tout cas être écartée de la lentille d'entrée de l'un et de l'autre desdits expandeurs de faisceau.

2

Il en résulte que, pour un même rapport d'expansion, les lentilles d'entrée et de sortie à mettre en œuvre peuvent être avantageusement des lentilles présentant des focales relativement longues, et donc des puissances relativement faibles, de l'ordre par exemple de 15 dioptries pour la lentille d'entrée.

De telles lentilles peuvent dès lors avantageusement se satisfaire de tolérances de fabrication plus larges, et être par exemple relevables d'une technique de surfaçage plus économique.

En outre, elles peuvent également avantageusement se satisfaire de tolérances de positionnement plus larges.

La réalisation de l'appareil de chirurgie ophtalmologique correspondant s'en trouve facilitée, et son coût réduit.

Son efficacité s'en trouve également améliorée, les faisceaux des lasers ayant globalement à traverser un nombre plus réduit d'interfaces air-verre ou verre-air, toujours générateurs de pertes.

Suivant un développement de l'invention, la lentille de sortie commune à l'expandeur de faisceau du laser à faisceau infrarouge et à l'expandeur de faisceau du laser à faisceau visible est formée par la lentille de focalisation de l'optique d'exploitation.

Cette lentille de sortie s'en trouve ainsi encore écartée d'autant par rapport aux lentilles d'entrée correspondantes, ce qui est avantageusement favorable à l'allongement recherché pour la focale de ces lentilles.

En outre, les réglages à effectuer s'en trouvent facilités.

En effet, la lentille de focalisation de l'optique d'utilisation étant disposée en aval du bras de transfert, et les faisceaux de chacun des lasers étant de ce fait divergents dans ce bras de transfert, et non plus cylindriques, il est plus facile d'assurer leur passage dans le bras de transfert, qui est cylindrique, le seul diaphragme rencontré étant la lentille de sortie commune aux deux expandeurs de faisceau et assurant la fonction de convergence.

Certes, dans la demande de brevet européen N° 0 030 210, il est mis en œuvre, d'une part, un laser de visée et, d'autre part, un laser de traitement.

Mais, il n'est associé un expandeur de faisceau qu'au seul laser de traitement, cet expandeur de faisceau étant constitué par deux lentilles, la première divergente, la seconde convergente, associées à celui-ci.

Par contre, aucun expandeur de faisceau n'est associé au laser de visée.

Seul se trouve en effet associé à celui-ci, non pas un expandeur de faisceau, mais un diviseur optique, qui, formé de prismes, en subdivise le faisceau en deux sous faisceaux.

En outre, l'expandeur de faisceau associé au laser de traitement et le diviseur optique associé au laser de visée n'ont, entre eux, aucun constituant commun.

Dans une variante de réalisation schématiquement représentée, il y a bien, dans cette demande de brevet européen N° 0 030 210, une lentille commune au laser de traitement et au laser de visée.

Mais cette lentille ne constitue pas la lentille de sortie de l'expandeur de faisceau associé au laser de traitement, dont la lentille convergente constitue au contraire, de manière usuelle, la lentille de sortie.

Il s'agit, en fait, d'une simple lentille de focalisation, dont il est admis ici qu'elle puisse être ainsi commune aux deux lasers.

Ici, suivant l'invention, il s'agit de lasers à chacun desquels il est associé un expandeur de faisceau.

De manière connue en soi, la superposition au faisceau du laser opératoire du faisceau du laser de visée, qui, suivant une caractéristique de l'invention, se fait au moins pour partie à l'intérieur même des expandeurs de faisceau correspondants, dans le tronçon que ces expandeurs de faisceau ont en commun, peut être obtenue en ajustant en conséquence la position des miroirs de renvoi interposés sur le faisceau du laser de visée.

Mais de préférence, suivant l'invention, elle est de manière très simple obtenue en interposant sur ce faisceau deux diasporamètres successifs, voire même deux simples lentilles successives, lesdites lentilles, dont chacune induit un prisme lorsqu'elle est excentrée, étant chacune montées mobiles dans leur plan.

Dans l'un et l'autre cas, les miroirs de renvoi mis en œuvre peuvent alors avantageusement être fixes.

Des dispositions semblables peuvent être adoptées lorsque, le laser auxiliaire de visée devant également servir de laser d'alignement pour le laser principal opératoire, une superposition des faisceaux de ces lasers est à assurer au sein même de la cavité de ce dernier.

Les caractéristiques et avantages de l'invention ressortiront d'ailleurs de la description qui va suivre, à titre d'exemple, en référence aux dessins schématiques annexés sur lesquels :

la figure 1 est un bloc diagramme en perspective d'un appareil de chirurgie ophtalmologique suivant l'invention ;

la figure 2 est une vue analogue à celle de la figure 1, pour une variante de réalisation de l'appareil de chirurgie ophtalmologique suivant l'invention.

Tel qu'illustré sur ces figures, l'appareil de chirurgie ophtalmologique suivant l'invention comporte, globalement, une unité à lasers 10 et une optique d'exploitation 11, pour un opérateur dont l'œil a été schématisé en 12, et pour un point opératoire schématisé en 13.

L'unité à lasers 10 comporte elle-même globalement un laser principal à faisceau infrarouge 15, au laser opératoire, un laser auxiliaire à faisceau visible 16, ou laser de visée, et un jeu de miroirs de renvoi, détaillé ci-après, propres à superposer lesdits faisceaux à l'entrée de l'optique d'exploitation 11.

De manière connue en soi, et tel que décrit notamment dans la demande de brevet européen EP-A-0 007 256 mentionnée ci-dessus, le laser opératoire 15 comporte par exemple de part et d'autre d'une tête de laser 18 contenant un barreau de YAG dopé au Néodyme, d'une part une cuve 19, à DYE ou autre solution d'absorbant saturable et miroir concave, et d'autre part un étalon de PEROT-FABRY 20.

Dans la forme de réalisation représentée, un diaphragme 21 est prévu entre la tête de laser 18 et la cuve 19.

L'axe Al du faisceau du laser opératoire 15 ainsi constitué se trouve successivement replié par un miroir de renvoi 22, qui, pour des raisons qui apparaîtront ci-après, est un miroir dichroïque, c'est-à-dire un miroir qui, réfléchissant pour le rayonnement infrarouge, laisse passer le rayonnement visible, un miroir de renvoi 23, et, à l'entrée de l'optique d'exploitation 11, un miroir de renvoi 24.

Ces miroirs 22, 23, 24 sont tous des miroirs mobiles.

Ils sont en pratique montés oscillants dans toutes les directions.

Parallèlement, le laser de visée 16 est par exemple un laser hélium-néon.

Par un miroir de renvoi 25, l'axe A2 de son faisceau est replié vers le miroir de renvoi 23, à travers le miroir de renvoi 22.

A compter du miroir 22, les faisceaux du laser opératoire 15 et du laser de visée 16 sont donc superposés.

L'optique d'exploitation 11 comporte, globalement, un bras de transfert 27, qui est formé de tronçons successivement articulés l'un à la suite de l'autre, et une lampe à fente 28, dont on a schématisé en 29 le microscope.

Elle comporte en outre sur son trajet optique, une lentille de focalisation 30.

Le bras de transfert 27 est équipé de miroirs de renvoi 32.

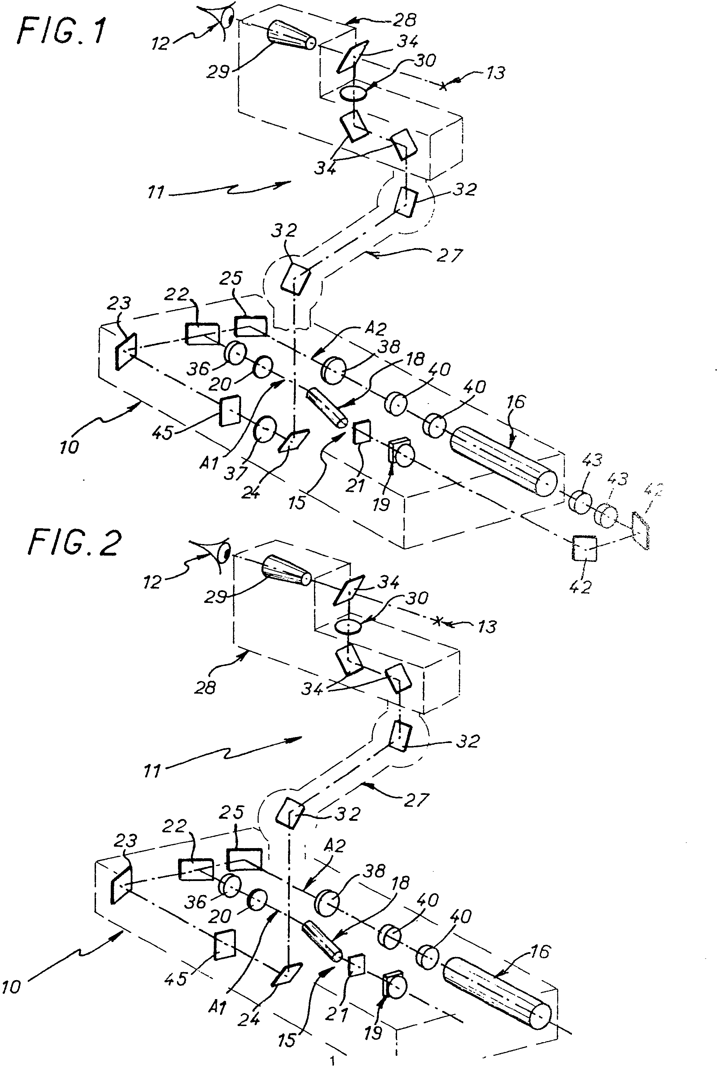

Tel que schématisé sur les figures 1, 2, il comporte ainsi, au moins, un miroir de renvoi 32, en regard du miroir de renvoi 24 formant le miroir de sortie de l'unité à laser 10, et un miroir de renvoi 32 à la base du pied vertical de la lampe à fente 28.

Tel que schématisé, celle-ci comporte également éventuellement, intérieurement, un certain nombre de miroirs de renvoi 34.

Ces dispositions sont bien connues par elles-mêmes, et ne faisant pas partie en soi de la présente invention, elles ne seront pas décrites plus en détail ici.

De manière également connue en soi, à chacun des lasers 15, 16 mis en œuvre il est associé un expandeur de faisceau au moins formé d'une lentille d'entrée et d'une lentille de sortie.

Suivant l'invention, l'expandeur de faisceau du laser opératoire 15 et celui du laser de visée 16 ont en commun leur lentille de sortie.

S'agissant du laser opératoire 15, l'expandeur de faisceau mis en œuvre comporte une lentille d'entrée 36, entre l'étalon de PEROT-FABRY 20 et le miroir de renvoi 22, et une lentille de sortie 37, entre les miroirs de renvoi 23 et 24.

En pratique, pour éviter un claquage optique dans l'air, la lentille d'entrée 36 est une lentille divergente ; par contre la lentille de sortie 37 est une lentille convergente.

Bien entendu, par lentille, on entend aussi bien ici une lentille unitaire qu'un groupe de lentilles accolées pour former conjointement l'équivalent optique d'une lentille unique.

Parallèlement, l'expandeur de faisceau du laser de visée 16 comporte une lentille d'entrée 38, entre sa tête de laser et le miroir de renvoi 25, et sa lentille de sortie est constituée par la lentille de sortie 37 précédente.

En pratique, la lentille d'entrée 38 est une lentille convergente.

Ainsi qu'on le notera, la lentille de sortie 37 commune aux expandeurs de faisceau des deux lasers peut être disposée au plus près du miroir de renvoi 24 formant le miroir de sortie de l'unité à lasers 10.

Elle est donc convenablement écartée de chacune des lentilles d'entrée 36 et 38 correspondantes.

Ainsi qu'on le notera également, les deux expandeurs de faisceau des lasers ont un tronçon commun, à compter du miroir de renvoi 22, et c'est au sein même de ce tronçon commun de ces expandeurs de faisceau que se fait la superposition des faisceaux desdits lasers à l'entrée de l'optique d'exploitation 11.

Pour les réglages nécessaires à cette superposition, il est prévu, suivant l'invention, sur l'axe A2 du faisceau du laser de visée 16, entre la tête de laser de celui-ci et la lentille d'entrée 38 de son expandeur de faisceau, deux diasporamètres 40 successifs.

Ainsi, le miroir de renvoi 25 peut avantageusement être un miroir fixe.

Suivant une variante de réalisation non représentée, chacun des diasporamètres 40 est lui-même remplacé par une simple lentille qui, induisant un prisme lorsqu'elle est excentrée, est montée mobile dans son plan, c'est-à-dire perpendiculairement à l'axe A2 du faisceau correspondant.

Quoi qu'il en soit, et de manière connue en soi, le miroir de renvoi 24 permet, parallèlement, de rattraper une erreur angulaire entre l'unité de laser 10 et l'optique d'exploitation 11.

Si désiré, et tel que schématisé en traits interrompus, le laser de visée 16 peut également servir de laser d'alignement pour le laser opératoire 15.

Il suffit, à cet effet, d'assurer une superposition de son faisceau au faisceau du laser opératoire 15 à l'intérieur même de la cavité de celui-ci.

Dans ce cas, deux miroirs de renvoi 42, disposés de l'autre côté de la tête de laser du laser de visée 16 par rapport à l'expandeur de faisceau associé à celui-ci, replient vers le laser opératoire 15 l'axe A2 du faisceau du laser de visée 16, avec interposition, comme précédemment, soit de deux diasporamètres 43 successifs, soit de deux lentilles successives dont chacune est montée mobile dans son plan.

Dans la variante de réalisation illustrée par la figure 2, la lentille de sortie commune à l'expandeur de faisceau du laser opératoire 15 et à l'expandeur de faisceau du laser de visée 16 est formée par la lentille de focalisation 30 de l'optique d'exploitation 11.

Autrement dit, la lentille 37 précédente est supprimée, et la lentille de focalisation 30 est établie de manière à assurer simultanément une double fonction de lentille de sortie pour les expandeurs de faisceau des lasers 15, 16, et de lentille de focalisation pour l'optique d'exploitation 11.

Dans tous les cas, et tel que schématisé en 45, un absorbant saturable est de préférence prévu entre les miroirs de renvoi 23, 24 de l'unité à lasers 10, pour éviter les super-radiances.

Les divers constituants mentionnés ci-dessus étant bien connus par eux-mêmes, et leurs réalisations relevant de l'homme de l'art, ils ne seront pas décrits plus en détail ici.

**Revendications**

1. Appareil de chirurgie ophtalmologique, du genre comportant un laser principal à faisceau infrarouge (15), ou laser opératoire, un laser auxiliaire à faisceau visible (16), ou laser de visée, un jeu de miroirs (22, 23, 24) propres à superposer lesdits faisceaux à l'entrée d'une optique d'exploitation (11), et, pour chacun de ceux-ci, un expandeur de faisceau au moins formé d'une lentille d'entrée (36, 38) et d'une lentille de sortie (37 ; 30), caractérisé en ce que l'expandeur de faisceau du laser à faisceau infrarouge (15) et l'expandeur de faisceau du laser à faisceau visible (16) ont en commun leur lentille de sortie (37 ; 30).

2. Appareil de chirurgie ophtalmologique suivant la revendication 1, dans lequel l'optique d'exploitation (11) comporte une lentille de focalisation (30), caractérisé en ce que la lentille de sortie commune à l'expandeur de faisceau du laser à faisceau infrarouge (15) et à l'expandeur de faisceau du laser à faisceau visible (16) est formée par la lentille de focalisation (30) de l'optique d'exploitation (11).

3. Appareil de chirurgie ophtalmologique suivant l'une quelconque des revendications 1 et 2, caractérisé en ce que la lentille d'entrée (36) de l'expandeur de faisceau du laser à faisceau infrarouge (15) est divergente.

4. Appareil de chirurgie ophtalmologique suivant l'une quelconque des revendications 1 à 3, caractérisé en ce que, pour superposition du faisceau du laser à faisceau visible (16) au faisceau du laser à faisceau infrarouge (15) à l'entrée de l'optique d'exploitation (11), il est interposé, sur le faisceau du laser à faisceau visible (16), entre ledit laser et son expandeur de faisceau, deux diasporamètres (40) successifs.

5. Appareil de chirurgie ophtalmologique suivant l'une quelconque des revendications 1 à 3, caractérisé en ce que, pour superposition du faisceau du laser à faisceau visible (16) au faisceau du laser à faisceau infrarouge (15) à l'entrée de l'optique d'exploitation (11), il est interposé, sur le faisceau du laser à faisceau visible (16), entre ledit laser et son expandeur de faisceau, deux lentilles successives, dont chacune est montée mobile dans son plan.

6. Appareil de chirurgie ophtalmologique suivant l'une quelconque des revendications 1 à 5, caractérisé en ce que, pour une superposition du faisceau du laser à faisceau visible (16) au faisceau du laser à faisceau infrarouge (15) à l'intérieur même de la cavité de celui-ci, il est interposé, sur le faisceau du laser à faisceau visible (16), entre ledit laser et ladite cavité, deux diasporamètres (43) successifs.

7. Appareil de chirurgie ophtalmologique suivant l'une quelconque des revendications 1 à 5, caractérisé en ce que, pour superposition du faisceau du laser à faisceau visible (16) au faisceau du laser à faisceau infrarouge (15) à l'intérieur même de la cavité de celui-ci, il est interposé, sur le faisceau du laser à faisceau visible (16), entre ledit laser et ladite cavité, deux lentilles successives, dont chacune est montée mobile dans son plan.

**Claims**

1. Apparatus for ophthalmological surgery, of the kind comprising a principal laser emitting an infrared beam (15), or operating laser, an auxiliary laser emitting a visible beam (16), or sighting laser, a set of mirrors (22, 23, 24) suitable for superimposing the said beams at the entrance of an operational optical system (11), and, for each one of the latter, a beam formed at least by an entrance lens (36, 38), and an exit lens (37 ; 30), characterised in that the beam expander of the laser emitting an infrared beam (15) and the beam expander of the laser emitting a visible beam (16) have their exit lens (37 ; 30) in common.

2. Apparatus for ophthalmological surgery according to Claim 1, wherein the operational optical system (11) comprises a focusing lens (30), characterised in that the exit lens common to the beam expander of the laser emitting an infrared beam (15) and to the beam expander of the laser emitting a visible beam (16) is formed by the focusing lens (30) of the operational optical system (11).

3. Apparatus for ophthalmological surgery according to any one of Claims 1 and 2, characterised in that the entrance lens (36) of the beam expander of the laser emitting an infrared beam (15) is diverging.

4. Apparatus for ophthalmological surgery according to any one of Claims 1 to 3, characterised in that, for superimposing the beam of the laser emitting a visible beam (16) on the beam of the laser emitting an infrared beam (15) at the entrance of the operational optical system (11), two successive rotating prisms (40) are interposed in the beam of the laser emitting a visible beam (16) between the said laser and its beam expander.

5. Apparatus for ophthalmological surgery according to any one of Claims 1 to 3, characterised in that, for superimposing the beam of the laser emitting a visible beam (16) on the beam of the laser emitting an infrared beam (15) at the entrance of the operational optical system (11), two successive lenses, each one of which is mounted so as to be movable in its plane, are interposed in the beam of the laser emitting a visible beam (16) between the said laser and its beam expander.

6. Apparatus for ophthalmological surgery according to any one of Claims 1 to 5, characterised in that, for superimposing the beam of the laser emitting a visible beam (16) on the beam of the laser emitting an infrared beam (15) within the actual cavity of the latter, two successive rotating prisms (43) are interposed in the beam of the laser emitting a visible beam (16) between the said laser and the said cavity.

7. Apparatus for ophthalmological surgery according to any one of Claims 1 to 5, characterised in that, for superimposing the beam of the laser emitting a visible beam (16) on the beam of the laser emitting an infrared beam (15) within the actual cavity of the latter, two successive lenses, each one of which is mounted to be movable in its plane, are interposed in the beam of the laser emitting a visible beam (16) between the said laser and the said cavity.

**Patentansprüche**

1. Vorrichtung zur ophthalmologischen Chirurgie, mit einem Hauptlaser oder Operationslaser (15) mit Infrarotstrahl, mit einem Hilfslaser oder Beobachtungslaser (16) mit sichtbarem Strahl, mit einem Satz von Spiegeln (22, 23, 24) zum Überlagern der Strahlen am Eingang einer Auswerteoptik (11) und mit einem Strahlenexpander für jeden dieser Strahlen, die wenigstens aus einer Eingangslinse (36, 38) und einer Ausgangslinse (37, 30) bestehen, dadurch gekennzeichnet, daß der Strahlenexpander des Lasers mit Infrarotstrahl (15) und der Strahlenexpander des Lasers mit sichtbarem Strahl (16) ihre Ausgangslinse (37, 30) gemeinsam haben.

2. Vorrichtung zur ophthalmologischen Chirurgie nach Anspruch 1, wobei die Auswerteoptik (11) eine Fokussierungslinse (30) aufweist, dadurch gekennzeichnet, daß die Ausgangslinse, die dem Strahlenexpander des Lasers mit Infrarotstrahl (15) und dem Strahlenexpander des Lasers mit sichtbarem Strahl (16) gemeinsam ist, von der Fokussierungslinse (30) der Auswerteoptik (11) gebildet wird.

3. Vorrichtung zur ophthalmologischen Chirurgie nach Anspruch 1 oder 2, dadurch gekennzeichnet, daß die Eingangslinse (36) des Strahlenexpanders des Lasers mit Infrarotstrahl (15) divergierend ist.

4. Vorrichtung zur ophthalmologischen Chirurgie nach einem der Ansprüche 1 bis 3, dadurch gekennzeichnet, daß zur Überlagerung des Strahls des Lasers mit sichtbarem Strahl (16) mit dem Strahl des Lasers mit Infrarotstrahl (15) am Eingang der Auswerteoptik (11) im Strahlengang des Lasers mit sichtbarem Strahl (16) zwischen dem Laser und seinem Strahlenexpander zwei aufeinanderfolgende Diasporameter (40) zwischengeschaltet sind.

5. Vorrichtung zur ophthalmologischen Chirurgie nach einem der Ansprüche 1 bis 3, dadurch gekennzeichnet, daß zur Überlagerung des Strahls des Lasers mit sichtbarem Strahl (16) mit dem Strahl des Lasers mit Infrarotstrahl (15) am Eingang der Auswerteoptik (11) im Strahlengang des Lasers mit sichtbarem Strahl (16) zwischen dem Laser und seinem Strahlenexpander zwei nacheinander angeordnete Linsen zwischengeschaltet sind, die beweglich in ihrer Ebene angeordnet sind.

6. Vorrichtung zur ophthalmologischen Chirurgie nach einem der Ansprüche 1 bis 5, dadurch gekennzeichnet, daß zur Überlagerung des Strahls des Lasers mit sichtbarem Strahl (16) mit dem Strahl des Lasers mit Infrarotstrahl (15) im Inneren dessen Hohlraums im Strahlengang des Lasers mit sichtbarem Strahl (16) zwischen dem Laser und dem Hohlraum zwei aufeinanderfolgende Diasporameter (43) zwischengeschaltet sind.

7. Vorrichtung zur ophthalmologischen Chirurgie nach einem der Ansprüche 1 bis 5, dadurch gekennzeichnet, daß zur Überlagerung des Strahls des Lasers mit sichtbarem Strahl (16) mit dem Strahl des Lasers mit Infrarotstrahl (15) in Inneren dessen Hohlraums im Strahlengang des Lasers mit sichtbarem Strahl (16) zwischen dem Laser und dem Hohlraum zwei aufeinanderfolgende Linsen zwischengeschaltet sind, die jeweils beweglich in ihrer Ebene angeordnet sind.

FIG.1

FIG.2

0 076 740